# EUROPÄISCHE PATENTSCHRIFT

(11) **EP 1 250 345 B1**
(45) Veröffentlichungstag und Bekanntmachung des Hinweises auf die Patenterteilung: **11.06.2003**
(21) Anmeldenummer: 01901165.9
(22) Anmeldetag: 18.01.2001
(51) Int. Cl.: C07H 21/00, C12Q 1/68

(54) **VERFAHREN, KIT UND DNA-SONDEN ZUM NACHWEIS EINER PILZ-SPEZIES IN KLINISCHEM MATERIAL**
METHOD, KIT AND DNA PROBES FOR DETECTING A FUNGAL SPECIES IN CLINICAL MATERIAL
PROCEDE, NECESSAIRE ET SONDES D'ADN POUR L'IDENTIFICATION D'UNE ESPECE DE CHAMPIGNON SE TROUVANT DANS UN MATERIAU CLINIQUE

(30) Priorität: 28.01.2000 DE 10003580
(43) Veröffentlichungstag der Anmeldung: 23.10.2002
(73) Patentinhaber: Eberhard-Karls-Universität Tübingen Universitätsklinikum, 72076 Tübingen (DE)
(72) Erfinder: LÖFFLER, Jürgen, 72766 Reutlingen (DE); EINSELE, Hermann, 72076 Tübingen (DE)
(74) Vertreter: Otten, Hajo, Dr.-Ing.
(86) Internationale Anmeldenummer: EP0100537
(87) Internationale Veröffentlichungsnummer: WO01055159

(56) Entgegenhaltungen:
- WO-A-97/07238
- US-A- 5 668 263
- US-A- 5 910 409

### SEQUENZPROTOKOLL

<110> Eberhards-Karls-Universität Tübingen Universitätsk
<120> DNA-Sonden zum Nachweis von Pilz-Spezies
<130> 5402P191
<140>
<141>
<160> 4
<170> PatentIn Ver. 2.1
<210> 1
<211> 28
<212> DNA
<213> Künstliche Sequenz
<220>
<223> Beschreibung der künstlichen Sequenz:DNA-Sonde für A.fumigatus
<400> 1
   tgaggttccc cagaaggaaa ggtccagc 28
<210> 2
<211> 24
<212> DNA
<213> Künstliche Sequenz
<220>
<223> Beschreibung der künstlichen Sequenz:DNA-Sonde für A.fumigatus
<400> 2
   gttcccccca cagccagtga aggc 24
<210> 3
<211> 25
<212> DNA
<213> Künstliche Sequenz
<220>
<223> Beschreibung der künstlichen Sequenz:DNA-Sonde für C. albicans
<400> 3
   tggcgaacca ggacttttac tttga 25
<210> 4
<211> 24
<212> DNA
<213> Künstliche Sequenz
<220>
<223> Beschreibung der künstlichen Sequenz:DNA-Sonde für C. albicans
<400> 4
   agcctttcct tctgggtagc catt 24

## Beschreibung

Die vorliegende Erfindung betrifft ein Verfahren zum Nachweis einer Pilz-Spezies in klinischem Material, mit den Schritten:
- Extraktion von Pilz-DNA aus klinischem Material,
- Bereitstellen eines DNA-Segmentes der extrahierten Pilz-DNA, und
- Nachweis der Pilz-Spezies durch Hybridisierung des DNA-Segmentes mit spezifischen DNA-Sonden.

Die Erfindung betrifft ferner bei den Verfahren einsetzbare DNA-Sonden sowie einen Kit, der die DNA-Sonden enthält.

Ein Verfahren der vorstehend genannten Art ist aus der WO 97/07238 bekannt. Diese Druckschrift offenbart ebenfalls Hybridisierungssonden, mit denen unter anderem Pilze der Spezies Candida albicans sowie Aspergillus fumigatus nachgewiesen werden können.

Invasive Pilzinfektionen haben als Hauptursache von Morbidität und Mortalität bei immunsupprimierten Patienten in den letzten Jahren eine erhebliche Bedeutung erlangt. Dies gilt z.B. für Patienten mit Knochenmark- oder Organtransplantation, Chemotherapie-Patienten, Aids-Patienten oder Patienten mit schweren Verbrennungen. Insbesondere bei hämatologischen Patienten ist die Aspergillose zur Haupttodesursache geworden.

Vor diesem Hintergrund ist eine frühe sichere Diagnose einer systemischen Pilzinfektion für eine geeignete und erfolgreiche Therapie unverzichtbar.

In der eingangs erwähnten WO 97/07238 wird zu diesem Zweck ein auf der Polymerasekettenreaktion (im folgenden: PCR) beruhendes Verfahren beschrieben, bei dem aus klinischem Material, vorzugsweise aus Vollblut, zunächst phagozytierte Pilzzellen extrahiert werden, aus denen dann die Pilz-DNA aufgereinigt wird. Mit Hilfe von Pilz-spezifischen Primern wird dann im Rahmen einer PCR ein DNA-Segment aus dem Pilz-Gen für die 18ssu-rRNA hochverstärkt. Dabei wird von der Erkenntnis ausgegangen, daß dieses Pilz-Gen bei verschiedenen Pilz-Stämmen und -Gattungen ein Sequenzsegment aufweist, das einerseits von zwei Bindungsregionen für Primer flankiert wird, die für alle Pilz-Stämme und -Gattungen identisch sind, das aber andererseits die Sequenz dieses Segmentes für die verschiedenen Pilz-Stämme und -Gattungen so verschieden ist, daß sie gleichzeitig zum Nachweis der einzelnen Pilz-Spezies und -Gattungen verwendet werden kann.

Das entsprechend hochverstärkte DNA-Segment wird dann sequentiell mit verschiedenen DNA-Sonden hybridisiert, die für die nachzuweisenden Pilz-Spezies jeweils spezifisch sind. Um eine erfolgreiche Hybridisierung nachzuweisen, werden die Sonden mit dem Transferase-Kit der Firma Boehringer, Mannheim, mit Digoxigenin markiert, wobei der Nachweis nach dem Southern Blot-Verfahren mit der üblichen Farbreaktion erfolgt.

Weitere Hybridisierungssonden, die bei dem bekannten Verfahren einsetzbar sind, sind in der DE 196 35 347 C1 beschrieben.

Obwohl das bekannte Verfahren unter Anwendung der bekannten DNA-Sonden sehr zuverlässig und mit hoher Empfindlichkeit arbeitet, besteht dennoch ein ständiger Bedarf an schneller und/oder einfacher durchzuführenden Verfahren. Ein Nachteil bei dem bekannten Verfahren sowie den bekannten Sonden besteht darin, daß das amplifizierte DNA-Segment sequentiell und/oder parallel in einzelnen Experimenten mit den jeweiligen DNA-Sonden inkubiert werden muß, wobei jeweils einzeln das Hybridisierungsergebnis zwar mit Standard-Verfahren, aber dennoch zeitaufwendig zu überprüfen ist.

In diesem Zusammenhang ist in den letzten Jahren ein Verfahren für quantitative PCR bekannt geworden, das auf dem sogenannten LightCycler™ von Roche Molecular Biochemicals durchgeführt werden kann. Mit diesem Verfahren ist es möglich, die Hochverstärkung der PCR-Produkte in Echtzeit und On-line Zyklus für Zyklus zu beobachten. Zu diesem Zweck werden der PCR-Lösung neben der Polymerase, den Nukleotiden, den Pufferlösungen und den Primern auch Hybridisierungssonden zugegeben, die spezifisch an die gewünschten PCR-Produkte binden. Dabei werden zwei Sequenzspezifische DNA-Sonden verwendet, die mit verschiedenen Farbstoffen markiert sind. Die Sequenzen der beiden Sonden sind so ausgewählt, daß sie so an die Zielsequenzen des verstärkten DNA-Segmentes hybridisieren, daß das 3'-Ende der einen Sonde dicht bei dem 5'-Ende der anderen Sonde liegt, wodurch die beiden Farbstoffe nahe zueinander gebracht werden. Der Donor-Farbstoff, z.B. Fluorescin, wird durch eine kurzwellige Lichtquelle angeregt und emittiert Fluoreszenzlicht bei einer etwas längeren Wellenlänge. Wenn sich die beiden Farbstoffe dicht beieinander befinden, regt die emittierte Energie den Akzeptor-Farbstoff an, der an der zweiten Hybridisierungssonde sitzt und ein Fluoreszenzlicht bei einer anderen Wellenlänge emittiert.

Dieser Energietransfer, der auch als Fluoreszenzresonanzenergietransfer (FRET) beteiligt wird, hängt sehr stark von dem Abstand zwischen den beiden Farbstoffmolekülen ab. Die Energie wird effizient nur dann transferiert, wenn die Moleküle sich in unmittelbarer Nähe (zwischen 1 und 5 Nukleotiden) befinden, wobei das Maß an Fluoreszenz direkt proportional zu der Menge an Ziel-DNA ist, die während des PCR-Prozesses erzeugt wird.

Aus dem Obigen ergibt sich, daß der Akzeptor-Farbstoff an der zweiten Hybridisierungssonde nur dann Fluoreszenzlicht emittiert, wenn beide Hybridisierungssonden an die Zielsequenz hybridisiert haben. Solange sich die beiden Hybridisierungssonden in Lösung befinden, ist nur eine sehr geringe Hintergrundfluoreszenz zu messen.

Durch die beiden Hybridisierungssonden wird darüber hinaus der PCR-Prozeß selbst nicht beeinträchtigt, denn die Hybridisierungssonden lösen sich jeweils wieder von der Ziel-DNA, wenn nach dem Anlagerungsschritt die Temperatur erhöht wird, um den nächsten komplementären Strang zu vervollständigen. Nach einer derartigen Replikationsrunde wird der entstandene Doppelstrang aufgeschmolzen, wobei sich im nächsten Anlagerungsschritt sowohl die Primer als auch die Hybridisierungssonden erneut an die Ziel-DNA anlagern können.

Vom Ergebnis her bedeutet dies, daß durch das soeben beschriebene Verfahren auf dem LightCycler™ die Zunahme an produzierter DNA durch eine Zunahme im Fluoreszenzsignal on-line verfolgt werden kann.

Eine Kombination der beiden insoweit beschriebenen Verfahren, also der PCR auf dem LightCycler™ mit dem Nachweisverfahren gemäß der WO 97/07238 würde gegenüber dem bisher bekannten Nachweisverfahren für Pilz-Spezies somit den Vorteil mitsichbringen, daß die Bereitstellung der DNA-Segmente aus dem 18ssu-rRNA-Gen sowie die Hybridisierung mit der DNA-Sonde in einer Lösung und in einer Reaktion erfolgen könnte, wodurch sich deutliche Zeitvorteile ergeben würden.

Vor diesem Hintergrund ist es Aufgabe der vorliegenden Erfindung, DNA-Sonden für das eingangs erwähnte Verfahren bereitzustellen, die eine Durchführung des bekannten Verfahrens mit einer Ausgestaltung wie bspw. auf dem LightCycler™ ermöglichen.

Erfindungsgemäß wird diese Aufgabe gelöst durch die Oligonukleotide SEQ ID-No: 1-4 aus dem beiliegenden Sequenzprotokoll. Dabei sind nicht nur die Oligonukleotide SEQ ID-No: 1-4 sondern auch Oligonukleotide, die mit einem dieser Oligonukleotide hybridisieren sowie Oligonukleotide, die mit einem mit einem derartigen Oligonukleotid hybridisierenden Oligonukleotid hybridisieren, von der Erfindung umfaßt. Nicht nur die Oligonukleotide mit den Sequenzen SEQ ID-No: 1-4 sondern auch deren komplementäre Sequenzen sowie leichte Modifikationen, die die Hybridisierung der Oligonukleotide mit dem DNA-Segment nicht nachteilig beeinträchtigen, lassen sich als DNA-Sonden verwenden.

Dabei sind Oligonukleotide mit der Sequenz SEQ ID-No: 1 und SEQ ID-No: 2 sowie entsprechende komplementäre und modifizierte Sequenzen zum Nachweis der Pilz-Spezies Aspergillus fumigatus und die Oligonukleotide mit den Sequenzen SEQ ID-No: 3 und SEQ ID-No: 4 sowie die komplementären und modifizierten Sequenzen zum Nachweis der Pilz-Spezies Candida albicans zu verwenden.

In einem spezifischen Ausführungsbeispiel ist das Oligonukleotid mit der Sequenz SEQ ID-No: 1 am 5'-Ende mit einem Akzeptor und das Oligonukleotid mit der Sequenz SEQ ID-No: 3 am 3'-Ende mit einem Donor-Farbstoff markiert. In entsprechender Weise ist das Oligonukleotid mit der Sequenz SEQ ID-No: 3 an seinem 5'-Ende mit einem Akzeptor- und das Oligonukleotid mit der Sequenz SEQ ID-No: 4 an seinem 3'-Ende mit einem Donor-Farbstoff markiert. Bei den jeweiligen komplementären Sequenzen sitzt der Akzeptor-Farbstoff am 3'- und der Donor-Farbstoff am 5'-Ende.

Die Erfinder der vorliegenden Anmeldung haben erkannt, daß es mit den insoweit beschriebenen DNA-Sonden möglich ist, in einem einzigen PCR-Versuch z.B. auf dem LightCycler™ Aspergillus fumigatus und Candida albicans nachzuweisen. Sofern die Akzeptor-Farbstoffe der DNA-Sonden für Aspergillus fumigatus und Candida albicans unterschiedlich sind, lassen sich in einem einzigen Versuch beide Pilz-Spezies nachweisen bzw. gegeneinander diskriminieren und quantifizieren.

Die neuen DNA-Sonden sind jedoch nicht nur für den LightCycler™ einsetzbar, sie ermöglichen auch bei einer "konventionellen" PCR einen schnelleren und einfacheren Nachweis der jeweiligen Pilz-Spezies als dies im Stand der Technik möglich war. In diesem Zusammenhang ist es nämlich lediglich erforderlich, nach der Hochverstärkung der DNA-Segmente die entsprechenden DNA-Sonden hinzuzugeben und dann in einem Fluorimeter zu vermessen, ob bei der Anregungswellenlänge des Donor-Farbstoffes ein Fluoreszenzsignal des oder der Akzeptor-Farbstoffe erkennbar ist. Auch hier ist eine Diskriminierung zwischen Aspergillus fumigatus und Candida albicans möglich.

Vor diesem Hintergrund wird die der Erfindung zugrundeliegende Aufgabe bei dem eingangs erwähnten Verfahren dadurch gelöst, daß als DNA-Sonden eine oder mehrere der erfindungsgemäßen Oligonukleotide verwendet werden, wobei vorzugsweise das DNA-Segment mittels einer PCR in für den Nachweis ausreichender Menge bereitgestellt wird.

Natürlich sind die neuen DNA-Sonden auch für Nachweisverfahren einsetzbar, bei denen die DNA-Segmente nicht mittels PCR sondern z.B. durch Klonierung oder andere Verfahren bereitgestellt werden.

In diesem Zusammenhang betrifft die Erfindung auch einen Kit zum Nachweis einer Pilz-Spezies, wobei zumindest eines der erfindungsgemäßen Oligonukleotide als DNA-Sonde in diesem Kit enthalten ist.

Weitere Vorteile ergeben sich aus der nachstehenden Beschreibung. Es versteht sich, daß die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Die folgenden Beispiele illustrieren das gesamte Verfahren zum Nachweis von Pilz-Spezies in klinischem Material.

### Beispiel 1: DNA-Extraktion

Die DNA-Extraktion erfolgt so wie beschrieben in Löffler et al., Med. Mycol. 36 (1998), Seiten 275-279:

5 ml EDTA-anticoaguliertes Blut wird zur Lyse der Erythrozyten in 15 ml einer hypertonen Lösung (10 mM Tris, 5 mM MgCl₂, 10 mM NaCl) inkubiert. Daraufhin folgt eine Lyse der Leukozyten in 1 ml Lysespuffer (10 mM Tris, 10 mM EDTA, 50 mM Nacl, 0,2 % SDS, 200 µg/ml Proteinase K). Nach entsprechender Zentrifugation befinden sich im Sediment jetzt die freigesetzten, phagozytierten Pilzzellen.

Das Sediment wird in 500 µl Puffer aufgenommen, der rekombinante Lyticase (1 U/100 µl) enthält, und für 45 min bei 37°C inkubiert, um Sphaeroblasten zu erzeugen. Der Puffer enthält neben Lyticase 50 mM Tris, 1 mM EDTA und 0,2 % β-Mercaptoethanol.

Lyse des Sphaeroblasten und Proteinpräzipitation erfolgt unter Verwendung des QIAmp Tissue Kit von Qiagen, Hilden, Deutschland, gemäß dem Protokoll des Herstellers.

Die eluierte DNA wird in 100 µl des Elutionspuffers aufgenommen und unmittelbar für die PCR verwendet oder bei -80°C gelagert.

### Beispiel 2: PCR auf dem LightCycler™

Mit Hilfe von zwei Pilz-spezifischen Primern, die bereits in der eingangs erwähnten WO 97/07238 beschrieben sind, wird jetzt ein DNA-Segment der extrahierten DNA auf den LightCycler™ amplifiziert und gleichzeitig nachgewiesen bzw. quantifiziert.

Die PCR erfolgt dabei in Glaskapillaren, die durch diese umströmende Luft auf die entsprechenden Reaktionstemperaturen gebracht werden. Die bereits erwähnten Primer (5'-ATT GGA GGG CAA GTC TGG TG und 5'-CCG ATC CCT AGT CGG CAT AG) binden an konservierte Bereiche des 18ssu-rRNA-Pilzgens und sorgen für die Amplifikation eines ca. 500 Basenpaare langen DNA-Segmentes, das mit Hilfe von zwei DNA-Sonden, die mit unterschiedlichen Farbstoffen markiert sind, nachgewiesen werden kann.

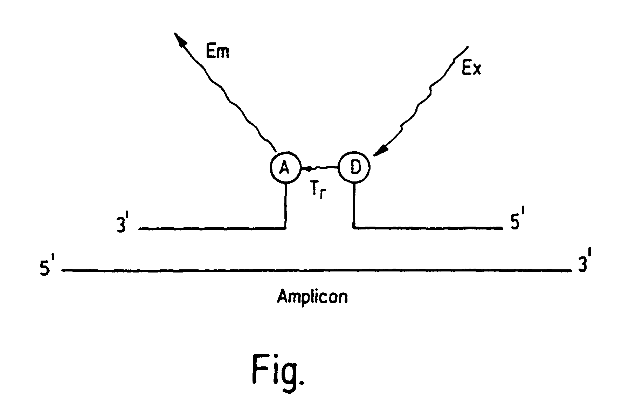

In Fig. 1 ist ein derartiges DNA-Segment dargestellt, das dort mit Amplicon bezeichnet ist.

Oberhalb des Amplicons sind zwei DNA-Sonden gezeigt, von denen die linke Sonde an ihrem 5'-Ende mit einem Akzeptor-Farbstoff A und die rechte Sonde an ihrem 3'-Ende mit einem Donor-Farbstoff D markiert ist. Die Sequenzen der beiden DNA-Sonden sind so ausgewählt, daß die Farbstoffe A und D nur ein bis fünf Nukleotide voneinander entfernt sind.

Wird jetzt der Farbstoff D bei einer entsprechenden Wellenlänge Ex angeregt, so emittiert er Licht auf einer Wellenlänge Tr, das zu einer Anregung des Farbstoffes A führt, der daraufhin auf einer Wellenlänge Em emittiert. Wenn aufgrund einer Anregung bei der Wellenlänge Ex eine Emission der Wellenlänge Em detektiert werden kann, bedeutet dies folglich, daß beide DNA-Sonden an das Amplicon hybridisiert sind, wodurch dieses Amplicon in der Reaktionslösung nicht nur nachgewiesen sondern auch quantifiziert werden kann, denn die Intensität des Fluoreszenzsignales bei der Wellenlänge Em steigt mit der Menge an in der Lösung befindlichem Amplicon an. Durch Rückrechnung auf den Startpunkt der PCR-Reaktion kann aus dem exponentiellen Verlauf des Fluoreszenzsignales über der Zahl der Zyklen in an sich bekannter Weise auf die Menge an ursprünglichem Amplicon in der Ausgangslösung bzw. der extrahierten DNA rückgeschlossen werden. Dazu wird ein externer Standard genomischer Pilz-DNA verwendet, die in Verdünnungsreihen mit gemessen wird.

Die PCR-Lösung enthielt Taq-Polymerase, LightCycler™ Hybridization 1 x Reaktionspuffer, dNTP-Mix, 3 mM MgCl₂ und 12,5 pmol Primer. Für die Amplicondetektion wurde der LightCycler™-DNA Master Hybridization Probes Kit nach den Angaben des Herstellers verwendet.

Zum Nachweis der Pilz-Spezies Aspergillus fumigatus wurden die folgenden DNA-Sonden eingesetzt:
5'-TGA GGT TCC CCA GAA GGA AAG GTC CAG C (SEQ ID-No: 1), am 5'-Ende markiert mit dem Akzeptor-Farbstoff LightCycler™-Red 640, und
5'-GTT CCC CCC ACA GCC AGT GAA GGC (SEQ ID-No: 2), am 3'-Ende mit dem Donor-Farbstoff Fluorescin markiert.

Für den Nachweis von Candida albicans wurden die folgenden DNA-Sonden eingesetzt:
5'-TGG CGA ACC AGG ACT TTT ACT TTG A (SEQ ID-No: 3), am 5'-Ende markiert mit LightCycler™-Red 640, und
5'-AGC CTT TCC TTC TGG GTA GCC ATT (SEQ ID-No: 4), am 3'-Ende mit Fluorescin markiert.

32 Proben wurden parallel verarbeitet mit jeweils 45 Zyklen von Denaturierung (1 sec bei 95°C), Annealing (15 sec bei 62°C) und enzymatische Kettenverlängerung (25 sec bei 72°C). Der PCR-Lauf benötigte 45 min.

### Beispiel 3: Ergebnisse

Die Empfindlichkeit des Verfahrens gemäß Beispiel 1 und 2 erwies sich als genauso groß wie bei dem bekannten Verfahren gemäß WO 97/07238, es konnten 5 CFU/ml ausgesäter Pilzzellen von Candida albicans bzw. Aspergillus fumigatus nachgewiesen werden. Der Test zeigte eine hohe Reproduzierbarkeit von 96-99 % und war linear in einem Bereich zwischen 10¹ und 10⁴ Konidien.

Versuche mit klinischem Material von Patienten mit hämatologischen Malignitäten und histologisch nachgewiesener invasiver Pilzinfektion waren ebenfalls erfolgreich. Fünf von neun positiven Proben zeigten eine Pilzlast zwischen 5 CFU/ml und 10 CFU/ml, zwei der neun Proben von zwischen 10 CFU/ml und 100 CFU/ml und die beiden letzten Proben von mehr als 100 CFU/ml.

Wenn die Sonde SEQ ID-No: 1 oder SEQ ID-No: 3 mit einem bei einer anderen Wellenlänge emittierenden Farbstoff, z.B. LightCycler™-Red 705 markiert wird, können Candida albicans und Aspergillus fumigatus in einer einzigen Reaktion nachgewiesen werden, indem Em sowohl bei 640 nm als auch bei 705 nm gemessen wird.

## Patentansprüche

1. Oligonukleotid mit der Nukleotidsequenz SEQ ID-No: 1 aus dem beiliegenden Sequenzprotokoll:

2. Oligonukleotid mit der Nukleotidsequenz SEQ ID-No: 2 aus dem beiliegenden Sequenzprotokoll:

3. Oligonukleotid mit der Nukleotidsequenz SEQ ID-No: 3 aus dem beiliegenden Sequenzprotokoll:

4. Oligonukleotid mit der Nukleotidsequenz SEQ ID-No: 4 aus dem beiliegenden Sequenzprotokoll:

5. Oligonukleotid, das mit einem Oligonukleotid gemäß einem der Ansprüche 1 bis 4 hybridisiert.

6. Oligonukleotid, das mit einem Oligonukleotid gemäß Anspruch 5 hybridisiert.

7. Verwendung der Oligonukleotide aus den Ansprüche 1 und 2 zum Nachweis von Aspergillus fumigatus.

8. Verwendung der Oligonukleotide aus den Ansprüche 3 und 4 zum Nachweis von Candida albicans.

9. Verfahren zum Nachweis einer Pilz-Spezies in klinischem Material, mit den Schritten:
- Extraktion von Pilz-DNA aus dem klinischem Material,
- Bereitstellen eines DNA-Segmentes der extrahierten Pilz-DNA, und
- Nachweis der Pilz-Spezies durch Hybridisierung des Pilz-Segmentes mit spezifischen DNA-Sonden,
**dadurch gekennzeichnet, daß** als DNA-Sonden eine oder mehrere der Oligonukleotide gemäß einem der Ansprüche 1 bis 6 verwendet werden.

10. Verfahren nach Anspruch 9, **dadurch gekennzeichnet, daß** das DNA-Segment mittels einer PCR in für den Nachweis ausreichender Menge bereitgestellt wird.

11. Kit zum Nachweis einer Pilz-Spezies, **dadurch gekennzeichnet, daß** zumindest eines der Oligonukleotide gemäß einem der Ansprüche 1 bis 6 als DNA-Sonde enthalten ist.

## Claims

1. An oligonucleotide having the nucleotide sequence SEQ ID NO: 1 from the attached sequence listing:

2. An oligonucleotide having the nucleotide sequence SEQ ID NO: 2 from the attached sequence listing:

3. An oligonucleotide having the nucleotide sequence SEQ ID NO: 3 from the attached sequence listing:

4. An oligonucleotide having the nucleotide sequence SEQ ID NO: 4 from the attached sequence listing:

5. An oligonucleotide which hybridizes with an oligonucleotide as claimed in any of claims 1 to 4.

6. An oligonucleotide which hybridizes with an oligonucleotide as claimed in claim 5.

7. The use of the oligonucleotides of claims 1 and 2 for detecting Aspergillus fumigatus.

8. The use of the oligonucleotides of claims 3 and 4 for detecting Candida albicans.

9. A method for detecting a fungal species in clinical material, comprising the steps:
- extracting fungal DNA from the clinical material,
- providing a DNA segment of the extracted fungal DNA, and
- detecting the fungal species by hybridizing the fungal segment with specific DNA probes,
**characterized in that** the DNA probes used are one or more of the oligonucleotides as claimed in any of claims 1 to 6.

10. The method of claim 9, **characterized in that** the DNA segment is provided by means of a PCR in an amount sufficient for detection.

11. A kit for detecting a fungal species, **characterized in that** it comprises at least one of the oligonucleotides as claimed in any of claims 1 to 6 as DNA probe.

## Revendications

1. Oligonucléotide ayant la séquence nucléotidique SEQ ID n° 1 provenant du protocole de séquençage ci-joint :

2. Oligonucléotide ayant la séquence nucléotidique SEQ ID n° 2 provenant du protocole de séquençage ci-joint :

3. Oligonucléotide ayant la séquence nucléotidique SEQ ID n° 3 provenant du protocole de séquençage ci-joint :

4. Oligonucléotide ayant la séquence nucléotidique SEQ ID n° 4 provenant du protocole de séquençage ci-joint :

5. Oligonucléotide, qui est hybridé avec un oligonucléotide selon l'une des revendications 1 à 4.

6. Oligonucléotide, qui est hybridé avec un oligonuclétide selon la revendication 5.

7. Utilisation des oligonucléotides selon les revendications 1 et 2, pour la détection d'*Aspergillus fumigatus.*

8. Utilisation des oligonucléotides selon les revendications 3 et 4, pour la détection de *Candida albicans*.

9. Procédé de détection d'une espèce fongique dans du matériau clinique, comprenant les étapes consistant à :
- extraire l'ADN fongique du matériau clinique ;
- mettre à disposition un segment d'ADN de l'ADN fongique extraite;
- détecter l'espèce fongique par hybridation du segment fongique avec des sondes ADN spécifiques,
**caractérisé en ce que** l'on peut utiliser comme sondes ADN un ou plusieurs des oligonucléotides selon l'une des revendications 1 à 6.

10. Procédé selon la revendication 9, **caractérisé en ce que** le segment d'ADN est mis à disposition, au moyen d'une PCR, en quantité suffisante pour la détection.

11. Trousse pour détecter une espèce fongique, **caractérisée en ce que** au moins l'un des oligonucléotides selon l'une des revendications 1 à 6 est contenu en tant que sonde ADN.
